# EUROPEAN PATENT APPLICATION

(11) **EP 2 269 652 A1**
(43) Date of publication of application: **05.01.2011**
(21) Application number: 09727040.9
(22) Date of filing: 26.03.2009
(51) Int. Cl.: A61K 47/14, A61K 8/37, A61K 8/42, A61K 9/10, A61K 9/70, A61Q 19/00

(54) **COMPOSITION FOR EXTERNAL APPLICATION TO SKIN**

(30) Priority: 31.03.2008 JP 2008090895
(71) Applicant: Rohto Pharmaceutical Co., Ltd., Ikuno-ku Osaka-shi Osaka 544-8666 (JP)
(72) Inventor: HIRAYAMA, Yoshiyuki, Osaka-shi Osaka 544-8666 (JP)
(74) Representative: Raynor, Stuart Andrew
(86) International application number: PCT/JP2009/056081
(87) International publication number: WO 2009/123003

(57) **Abstract**

An object of the present invention is to provide an external composition for skin, which can give good warmth without inducing irritation or pain and whose sustainability of warmth is good enough. The external composition for skin of the present invention is **characterized in that** it contains (A) an agent for giving warmth and (B) at least one selected from the group consisting of fatty acid esters of aliphatic polyhydric alcohols and aromatic carboxylic acid esters of lower alcohols or aliphatic polyhydric alcohols.

## Description

### Technical Field

The present invention relates to an external composition for skin. More specifically, the present invention relates to an external composition for skin which gives warmth.

### Background Art

Conventionally, for the purpose of giving warmth, antiinflammatory and antipruritic actions, powdered capsicum, capsicum tincture, capsicum extract, ginger extract, capsaicin, nonanoic acid vanillylamide, methyl salicylate, or the like is contained in external preparation for skin (Patent Documents 1 and 2). Since containing of such a warmth ingredient may cause irritation, a rash or the like on the skin, a containing amount is preferably kept at low level. However, the low containing amount has a problem that satisfactory warmth cannot be given, sustainability of warmth becomes insufficient or the like.

As a method for resolving such a problem, the following methods have been developed:
(1) a method of using several warmth ingredients in combination (Patent Document 3);
(2) a method of using an adhesive skin patch in which an adhesive agent and a release film are laminated on a support which is a specific woven cloth, the adhesive agent containing an agent for giving warmth and an oil ingredient (e.g., essential oil containing monoterpenoid, or the like) (Patent Document 4); and
(3) a method of using a fatty acid ester (e.g., diisopropyl adipate, isopropyl myristate or the like) as a warmth-enhancing component (Patent Document 5).

However, the advantages of these methods are still not satisfactory.
Patent Document 1: JP-A-2006-206471
Patent Document 2: JP-A-2000-204046
Patent Document 3: JP-A-2000-026267
Patent Document 4: JP-A-2002-193794
Patent Document 5: JP-A-2003-206239

### Disclosure of Invention

### Problems to be Solved by the Invention

It is an object of the present invention to provide an external composition for skin which can give good warmth without inducing irritation or pain and whose sustainability of warmth is good enough.

### Means for Solving the Problems

As a result of investigation of combinations of a warmth ingredient and various ingredients in order to resolve the above-mentioned problems, the present inventor has found that it is possible to produce an external composition for skin which can give good warmth continuously by mixing a specific ester compound. Thus, the inventor completed the present invention.

That is to say, the gist of the present invention is as follows.
An external composition for skin comprising:
(A) an agent for giving warmth; and
(B) at least one (simply referred to as "ingredient (B)" hereinafter) selected from the group consisting of fatty acid esters of aliphatic polyhydric alcohols (excluding glyceryl tri-2-ethylhexanoate) and aromatic carboxylic acid esters of lower alcohols or aliphatic polyhydric alcohols.

The agent for giving warmth (A) preferably contains at least one selected from the group consisting of capsaicinoid, N-acylvanillylamide and vanillyl alcohol alkyl ethers.

The ingredient (B) preferably contains an aliphatic polyhydric alcohol fatty acid ester (excluding glyceryl tri-2-ethylhexanoate).
The ingredient (B) more preferably contains a propylene glycol fatty acid ester.

The ingredient (B) still more preferably contains at least one selected from the group consisting of propylene glycol monocaprylate, propylene glycol dicaprylate and propylene glycol didecanoate.

The agent for giving warmth (A) preferably contains capsaicinoid.
It is possible to produce skin external preparations by forming the external composition for skins of the present invention to various pharmaceutical formulations.

In an adhesive skin patch in which an adhesive layer comprising the aforementioned external composition for skin of the present invention is formed on a support, the advantage of the present invention is significantly exhibited.
In an adhesive skin patch having a support, a layer (1) containing the aforementioned external composition for skin of the present invention and an adhesive layer, the advantage of the present invention is also significantly exhibited.

In the adhesive skin patch, the support, the layer (1), and the adhesive layer are preferably laminated in this order.
Other preferred pharmaceutical formulations to which the external composition for skin of the present invention can be formed include a cream.

### Advantages of the Invention

The present invention provides an external composition for skin which can give good warmth without inducing irritation or pain and whose sustainability of warmth is good enough.Brief Description of Drawings

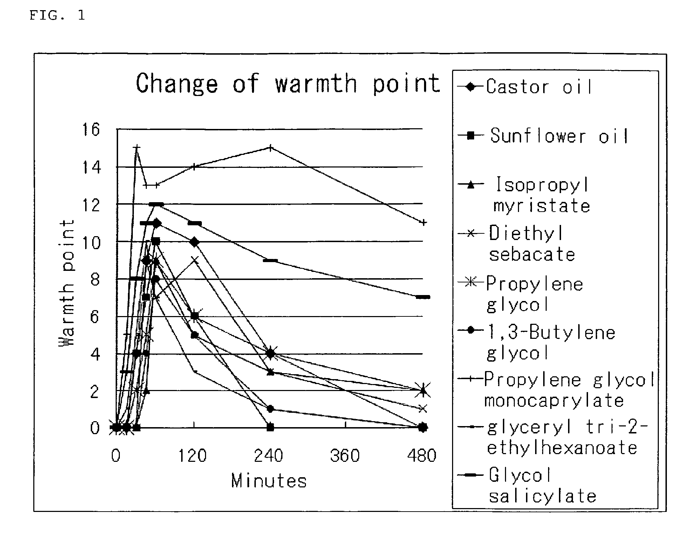

[Fig. 1] Fig. 1 shows change over time in warmth points in Test Example 1. The nine polygonal lines shown in Fig. 1 indicate Example 1 by + markers, Example 2 by landscape rectangular markers, Comparative Example 1 by rhombic markers, Comparative Example 2 by square markers, Comparative Example 3 by circular markers, Comparative Example 4 by asterisk markers, Comparative Example 5 by triangular markers, Comparative Example 6 by cross markers and Comparative Example 7 by smallest rectangular markers among the markers on the nine polygonal lines.

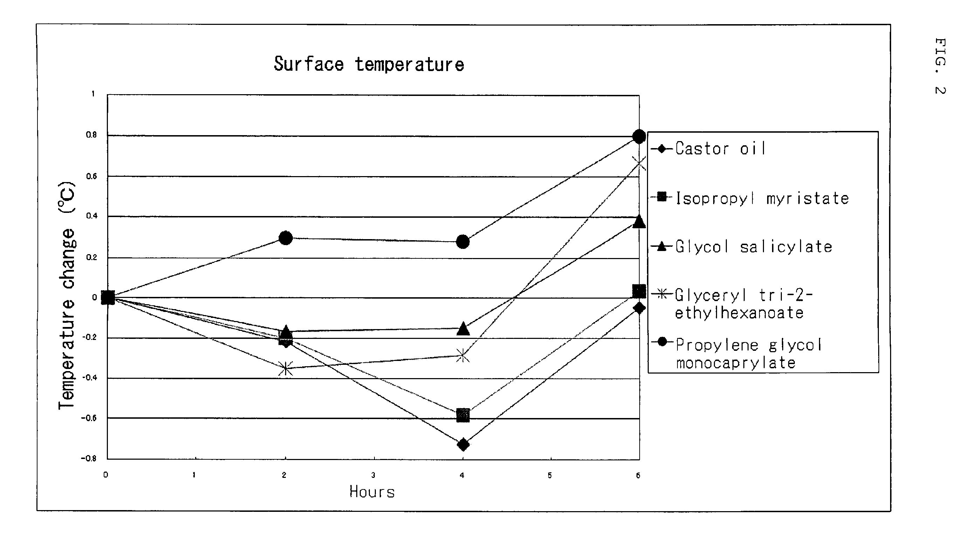

[Fig. 2] Fig. 2 shows change in temperature of skin surface in Test Example 2. The five polygonal lines shown in Fig. 2 indicate Example 1 by circular markers, Example 2 by triangular markers, Comparative Example 1 by rhombic markers, Comparative Example 5 by square markers and Comparative Example 7 by asterisk markers.

### Best Mode for Carrying Out the Invention

The present invention is described in detail below.

### [External composition for skin]

An external composition for skin of the present invention is characterized in that it contains (A) an agent for giving warmth and (B) at least one selected from the group consisting of fatty acid esters of aliphatic polyhydric alcohols (excluding glyceryl tri-2-ethylhexanoate) and aromatic carboxylic acid esters of lower alcohols or aliphatic polyhydric alcohols. Each of the ingredients is described below.

### <(A) Agent for giving warmth>

The external composition for skin of the present invention contains the agent for giving warmth (A). Examples of the agent for giving warmth (A) include capsaicinoid, N-acylvanillylamide, vanillyl alcohol alkyl ether, benzyl nicotinate, pelargonic acid, β-butoxyethyl nicotinate, capsicoside, and capsanthin.

Among them, capsaicinoid, N-acylvanillylamide and vanillyl alcohol alkyl ether are preferred as the agent for giving warmth (A).
As the capsaicinoid, capsaicin, dihydrocapsaicin, nordihydrocapsaicin, homodihydrocapsaicin and the like are preferred. The external composition for skin of the present invention may contain the capsaicinoid as a capsicum extract, a capsicum tincture or powdered capsicum.

An acyl group in the N-acylvanillylamide is a substituent group represented by R₁CO- wherein R₁ represents a monovalent hydrocarbon group which may be branched or substituted. The number of carbon atoms of the monovalent hydrocarbon group is not particularly limited as long as the advantage of the present invention is exhibited. As the acyl group, an acyl group having 6 to 12 carbon atoms such as a nonanoyl group is preferred. As the N-acylvanillylamide, nonylic acid vanillylamide is particularly preferred.

An alkyl in the vanillyl alcohol alkyl ether represents an usual alkyl, i.e., a monovalent group produced by removing one hydrogen atom from an aliphatic hydrocarbon. The alkyl group may be substituted. As the alkyl group, an alkyl group having 1 to 8 carbon atoms which may be branched such as a methyl group, an ethyl group, a propyl group, an isopropyl group, a n-butyl group, a sec-butyl group, a tert-butyl group, an isobutyl group, a pentyl group, a hexyl group, a heptyl group and an octyl group is preferred.

As the vanillyl alcohol alkyl ether, vanillyl alcohol butyl ether is particularly preferred.
These agents for giving warmth (A) can be used alone or in combination of two or more kinds thereof.

The agent for giving warmth (A) preferably contains capsaicinoid from the viewpoint that good warming effect and warming effect sustained by the ingredient (B) are significantly exhibited. In particular, as the capsaicinoid, capsaicin, dihydrocapsaicin, nordihydrocapsaicin, homodihydrocapsaicin, a mixture thereof (for example, capsaic.in USP or the like), and an extract, tincture and powder containing these capsaicinoid (for example, capsicum USP, capsicum oleoresin USP, capsicum extract-20, capsicum extract-Z and the like) are preferred. Capsaicin USP (a capsaicin content of 55% by weight or more, a total content of capsaicin and dihydrocapsaicin of 75% or more, a content of other capsaicinoid of 15% or less (all calculated in terms of dried products thereof)) is particularly preferred.

The content of the agent for giving warmth (A) in the external composition for skin of the present invention is generally 0.0001 to 5% by weight, preferably 0.001 to 1% by weight and more preferably 0.01 to 0.5% by weight based on the total external composition for skin (100% by weight). When the content of the agent for giving warmth (A) is less than 0.0001% by weight, the sufficient warmth effect may not be given, while when the content exceeds 5% by weight, irritation, rash or the like may occur on the skin. When the agent for giving warmth (A) is contained as an extract or powder in the external composition for skin of the present invention, "the content of the agent for giving warmth (A)" represents the amount of the agent for giving warmth (A) contained as an active ingredient in the extract or powder. For example, when the agent for giving warmth (A) is capsaicinoid and contained as an extract in the external composition for skin, "the content of the agent for giving warmth (A)" is the amount of capsaicinoid.

### <Ingredient (B)>

The external composition for skin of the present invention contains (B) at least one selected from the group consisting of fatty acid esters of aliphatic polyhydric alcohols (excluding glyceryl tri-2-ethylhexanoate) and aromatic carboxylic acid esters of lower alcohols or aliphatic polyhydric alcohols. With glyceryl tri-2-ethylhexanoate, the advantage of the present invention is not exhibited.

The external composition for skin of the present invention contains the ingredient (B) and thus can give good warmth in significantly sustained way (for a long period of time, generally 4 hours or more, preferably 6 hours or more, particularly preferably 8 hours or more, and usually 10 hours or less). Patent Document 2 (JP-A-2000-204046) discloses an external preparation characterized in that it is obtained by mixing an antipruritic agent and a base, the base being prepared by mixing a polyhydric alcohol fatty acid ester, an oil, a lower alcohol and water. Examples of the oil which is mixed for antipruritic effect exhibited excellently even at a low concentration of the antipruritic agent include ester oils. Examples of the ester oils include propylene glycol monocaprylate, propylene glycol dicaprylate and the like. However, the effect of warmth and duration of the warmth of these oils are not verified.

As a result of investigation of various compounds including these compounds, the present inventor has found that specific ester compounds have the two effects below, and completed the present invention.
(1) The effect of significantly increasing the duration of the warmth-giving effect of the agent for giving warmth (A) quite different from the effect of the oil disclosed in Patent Document 2.
(2) The effect of significantly decreasing or eliminating an irritating sensation and pain of the agent for giving warmth (A).

That is to say, the present invention has been achieved on the basis of the finding that by using the agent for giving warmth (A) and the ingredient (B) in combination, warmth can be continuously given for a long time, and an irritating sensation and pain of the agent for giving warmth (A) are significantly decreased. The term "in combination" represents that both the agent for giving warmth (A) and the ingredient (B) are applied. Further, when the ingredient (B) is a specific compound, the surface temperature can be increased by using the agent for giving warmth (A) and the ingredient (B) in combination as described below.

### (Fatty acid ester of aliphatic polyhydric alcohol)

Aliphatic polyhydric alcohols in the fatty acid esters of aliphatic polyhydric alcohols represent hydrocarbons each of which has two or more hydroxyl groups and may be branched or substituted by a group other than a hydroxyl group. The numbers of carbon atoms of the hydrocarbons are not particularly limited as long as the advantage of the present invention is exhibited, but are generally 2 to 9, preferably 2 to 4, and particularly preferably 2 to 3.

From the viewpoint of extending the duration of the good warmth-giving effect without causing sensation of irritation or pain, preferred examples of the aliphatic polyhydric alcohols include glycerin, diglycerin, ethylene glycol, propylene glycol(1,2-propanediol), 1,3-propanediol, 1,2-butanediol, 1,3-butanediol, 1,4-butanediol, pentaerythritol, trimethylolpropane and 2-butyl-2-ethyl-1,3-propanediol. Propylene glycol is particularly preferred.

Fatty acids in the fatty acid esters of aliphatic polyhydric alcohols represent compounds represented by R₂COOH wherein R₂ represents a hydrocarbon group which may be branched or substituted. The number of carbon atoms of the hydrocarbon group is not particularly limited as long as the advantage of the present invention is exhibited. From the viewpoint of extending the duration of the good warmth-giving effect, preferred examples of the fatty acids include fatty acids having 7 to 20 carbon atoms which may be branched such as heptanoic acid, caprylic acid, decanoic acid, lauric acid, stearic acid, isostearic acid, capric acid and 2-ethylhexanoic acid. Caprylic acid and decanoic acid are more preferred, and caprylic acid is particularly preferred.

Preferred examples of the fatty acid esters of aliphatic polyhydric alcohols include propylene glycol monocaprylate, propylene glycol dicaprylate, propylene glycol didecanoate, diglyceryl triisostearate, propylene glycol dicaprate, pentaerythritol tetra-2-ethylhexanoate, trimethylolpropane tri-2-ethylhexanoate, trimethylolpropane triisostearate, glyceryl tricapryl-caprate and 2-butyl-2-ethyl-1,3-propanediol 2-ethylhexanoate. Propylene glycol monocaprylate, propylene glycol dicaprylate and propylene glycol didecanoate are more preferred, and propylene glycol monocaprylate is particularly preferred.

When the external composition for skin of the present invention contains a fatty acid ester of an aliphatic polyhydric alcohol as the ingredient (B), the composition can not only give warmth continuously but also further increase the surface temperature. By increasing the surface temperature, effects such as promotion of blood circulation and promotion of metabolism are expected.

### (Aromatic carboxylic acid esters of lower alcohols or aliphatic polyhydric alcohols)

Lower alcohols in the aromatic carboxylic acid esters of lower alcohols or aliphatic polyhydric alcohols are usually monovalent alcohols having 1 to 6 carbon atoms which may be branched. Examples of the lower alcohols include methanol, ethanol, propanol, isopropanol, n-butyl alcohol, sec-butyl alcohol, tert-butyl alcohol, isobutyl alcohol, pentyl alcohol and hexyl alcohol.

The aliphatic polyhydric alcohols are as described above. From the viewpoint of extending the duration of the good warmth-giving effect, ethylene glycol is preferred as an aliphatic polyhydric alcohol.
The aromatic carboxylic acids represent compounds wherein a carboxyl group is directly bonded to an aromatic ring of an aromatic hydrocarbon. Specific examples of the aromatic carboxylic acids include benzoic acid, phthalic acid, salicylic acid, anthranilic acid and nicotinic acid. From the viewpoint of extending the duration of the good warmth-giving effect, salicylic acid is preferred as an aromatic carboxylic acid.

As the aromatic carboxylic acid esters of lower alcohols or aliphatic polyhydric alcohols, glycol salicylate and methyl salicylate are preferred, and glycol salicylate is particularly preferred.

Regarding the ingredient (B), a fatty acid ester of an aliphatic polyhydric alcohol exhibits the strong effect of extending the duration of the good warmth-giving effect as compared with that of an aromatic carboxylic acid ester of a lower alcohol or aliphatic polyhydric alcohol, and further produces the effect of increasing the surface temperature of an applied part. Therefore, the ingredient (B) preferably contains a fatty acid ester of an aliphatic polyhydric alcohol.

The content of the ingredient (B) in the external composition for skin of the present invention is generally 0.01 to 15% by weight, preferably 0.1 to 5% by weight, and more preferably 0.5 to 3% by weight based on the total external composition for skin(100% by weight). Within this range, the effect of extending the duration of giving good warmth can be produced.

With respect to the blending ratio between the agent for giving warmth (A) and the ingredient (B) in the external composition for skin of the present invention, the amount of the ingredient (B) is generally 1 to 3,000 parts by weight, preferably 10 to 1,000 parts by weight, more preferably 20 to 500 parts by weight, still more preferably 20 to 100 parts by weight, and particularly preferably 20 to 50 parts by weight based on 1 part by weight of the agent for giving warmth (A). Within this range, the effect of extending the duration of giving good warmth can be produced.

As described above, the external composition for skin of the present invention contains the agent for giving warmth (A) and the ingredient (B) which is a specific ester compound. By inclusion of the ingredient (B), the warmth-giving effect of the agent for giving warmth (A) becomes sustained for a long time, and further an irritating sensation and pain caused by the agent for giving warmth (A) are significantly decreased or eliminated. If necessary, the external composition for skin of the present invention may contain other ingredients described below.

### <Other ingredients>

The external composition for skin of the present invention may contain a base, a surfactant, a thickening agent, a preservative, a pH adjuster, an antioxidant, a chelating agent, a stabilizer, an irritation-reducing agent, a coloring agent, a dispersing agent, a perfume, and the like within the quantitative and qualitative range that does not deteriorate properties such as storage stability or viscosity, and does not impair the advantages of the present invention.

Also, the external composition for skin of the present invention may contain an anti-inflammatory agent, a moisturizer, a vitamin, an astringent, an anti-wrinkle agent and the like in order to add other useful effects within quantitative and qualitative range that does not impair the advantages of the present invention.

These ingredients are not particularly limited, and any desired ingredients which are conventionally used and will be used in the future as ingredients for external composition for skins can be appropriately selected. The ingredients can be used alone or in combination of two or more kinds thereof. The blending amount of each of the ingredients is not particularly limited as long as the advantages of the present invention are exhibited. The blending amount can be appropriately determined on the basis of a probable pharmaceutically acceptable upper limit.

Although examples of each of the ingredients which can be arbitrarily contained are given below, the ingredients which can be arbitrarily contained are not limited to them.

### (Base)

Hydrocarbons such as paraffin, ozokerite, ceresin, hard fat, microcrystalline wax, squalane (synthetic and plant-derived), α-olefin oligomers, liquid paraffin, light isoparaffin, liquid isoparaffin, polyethylene powder, gelled hydrocarbons and vaseline;
fatty acids such as lauric acid, myristic acid, palmitic acid, stearic acid, behenic acid, isostearic acid, oleic acid and linoleic acid;
tri-fatty acid glycerides such as glyceryl tri-2-ethylhexanoate (trioctanoin);
polymerized silicones such as highly polymerized methylpolysiloxane, dimethylpolysiloxane, dimethylsiloxane/methyl(polyoxyethylene)siloxane/methyl (polyoxypropylene)siloxane copolymer, dimethylsiloxane/methyl(polyoxyethylene)siloxane copolymer, dimethylsiloxane/methyl(polyoxypropylene)siloxane copolymer, polyoxyethylene/methylpolysiloxane copolymer, poly(oxyethylene/oxypropylene)/methylpolysiloxane copolymer, dimethylsiloxane/methylcetyloxysiloxane copolymer, dimethylsiloxane/methylstearoxysiloxane copolymer, alkyl acrylate copolymer methylpolysiloxane esters, crosslinked methylpolysiloxane, crosslinked methylphenylpolysiloxane, crosslinked polyether-modified silicones, crosslinked alkyl polyether-modified silicones and crosslinked alkyl-modified silicones;
glycol acetates such as ethylene glycol monoacetate, ethylene glycol diacetate, triethylene glycol diacetate, hexylene glycol diacetate and 2-methyl-2-propene-1,1-diol diacetate;
glycol esters such as triethylene glycol divalerate, 2,2,4-trimethyl-1,3-pentanediol monoisobutyrate and 2,2,4-trimethyl-1,3-pentanediol diisobutyrate;
glycol acrylates such as ethylene glycol diacrylate, diethylene glycol diacrylate, propylene glycol monoacrylate, 2,2-dimethyl-trimethylene glycol diacrylate and 1,3-butylene glycol diacrylate;
glycol dinitrates such as ethylene glycol dinitrate, diethylene glycol dinitrate, triethylene glycol dinitrate and propylene glycol dinitrate;
ether compounds such as 2,2'-[1,4-phenylenedioxy]diethanol, dioxane and polyester of butylene glycol adipate;
lower alcohols such as ethanol and isopropanol;
higher alcohols such as cetanol, stearyl alcohol, behenyl alcohol, cetostearyl alcohol, 2-hexyldecanol, isostearyl alcohol, 2-octyl dodecanol, oleyl alcohol, decyl tetradecanol, myristyl alcohol and lauryl alcohol;
polyhydric alcohols such as ethylene glycol, propylene glycol, 1,3-butylene glycol, 1,2-pentanediol, 1,2-hexanediol and glycerin;
diethylene glycol alkyl ethers such as diethylene glycol monoethyl ether;
macrogol;
esters such as isopropyl myristate, octyldodecyl myristate, isopropyl palmitate and cetyl palmitate;
polyoxyethylene alkyl ethers such as polyoxyethylene behenyl ether; and
vegetable oils such as olive oil, eucalyptus oil, turpentine oil, castor oil, peppermint oil and safflower oil.

### (Surfactant)

Sorbitan fatty acid esters such as sorbitan monoisostearate, sorbitan monolaurate, sorbitan monopalmitate, sorbitan monostearate, diglycerol sorbitan penta-2-ethylhexylate and diglycerol sorbitan tetra-2-ethylhexylate;
polyglyceryl fatty acid esters such as polyglyceryl monoisostearate and polyglyceryl diisostearate;
hydrogenated castor oil derivatives such as polyoxyethylene hydrogenated castor oil 40, polyoxyethylene hydrogenated castor oil 50, polyoxyethylene hydrogenated castor oil 60 and polyoxyethylene hydrogenated castor oil 80;
polyoxyethylene sorbitan fatty acid esters such as polyoxyethylene (20) sorbitan monolaurate (polysorbate 20), polyoxyethylene (20) sorbitan monostearate (polysorbate 60) and polyoxyethylene (20) sorbitan monooleate (polysorbate 80),; and
polyoxyethylene glyceryl monococoate, glyceryl alkyl ethers, alkyl glucosides, polyoxyethylene cetyl ether, stearylamine, oleylamine and the like.

### (Thickening agent)

Cellulose polymers such as methyl cellulose, ethyl cellulose, carboxymethyl cellulose, hydroxyethyl cellulose, hydroxyethyl methyl cellulose, hydroxypropyl cellulose, hydroxypropyl methyl cellulose, carmellose sodium and stearoxy hydroxypropyl methyl cellulose;
vinyl polymers such as polyvinyl alcohol (partially saponified products thereof), polyvinylpyrrolidone, polyethylene glycol, carboxyvinyl polymer, polyvinyl methyl ether and ammonium N-acryloyldimethyltaurate/vinylpyrrolidone copolymer;
acrylic acid polymers such as sodium polyacrylate, partially neutralized polyacrylic acid and acrylic acid/alkyl methacrylate copolymers (such as Pemulen (registered trademark));
plant polymers such as gum arabic, tragacanth gum, galactan, guar gum, pectin, carrageenan, alginic acid, sodium alginate, propylene glycol alginate ester and locust bean gum;
microbial polymers such as xanthan gum, dextran and pullulan;
mucopolysaccharides such as chondroitin sulfuric acid, sodium chondroitin sulfate, hyaluronic acid and sodium hyaluronate; and
bentonite, kaoline, talc, dextrin fatty acid esters and the like.

### (Preservative)

Benzoic acid, sodium benzoate, dehydroacetic acid, sodium dehydroacetate, isobutyl paraoxybenzoate, isopropyl paraoxybenzoate, butyl paraoxybenzoate, ethyl paraoxybenzoate, propyl paraoxybenzoate, benzyl paraoxybenzoate, methyl paraoxybenzoate, phenoxyethanol and 1,2-hexanediol.

### (pH adjuster)

Inorganic acids such as hydrochloric acid, sulfuric acid, phosphoric acid, polyphosphoric acid and boric acid;
organic acids such as lactic acid, acetic acid, citric acid, tartaric acid, malic acid, succinic acid, sodium succinate, oxalic acid, gluconic acid, fumaric acid, propionic acid, acetic acid, aspartic acid, epsilon-aminocaproic acid, glutamic acid and aminoethylsulfonic acid;
gluconolactone;
ammonium acetate;
inorganic bases such as sodium hydrogencarbonate, sodium carbonate, potassium hydroxide, sodium hydroxide, calcium hydroxide and magnesium hydroxide; and
organic bases such as monoethanolamine, triethanolamine, diisopropanolamine, triisopropanolamine and lysine.

### (Antioxidant)

Butylhydroxyanisole, dibutylhydroxytoluene, sodium hydrogen sulfite, erythorbic acid and salts thereof, glutathione, glutathione peroxidase, glutathione-S-transferase, catalase, superoxide dismutase, thioredoxin, taurine, thiotaurine, hypotaurine and the like.

### (Chelating agent)

Ethylenediaminetetraacetic acid (edetic acid), ethylenediaminetetraacetates (sodium salt (sodium edetate: Japanese Pharmacopoeia, EDTA-2Na and the like), potassium salt and the like), phytic acid, gluconic acid, polyphosphoric acid, metaphosphoric acid and the like.

### (Anti-inflammatory agent)

Glycyrrhizic acid derivatives such as Glycyrrhiza extract, glycyrrhizic acid and dipotassium glycyrrhizinate, monoammonium glycyrrhizinate; glycyrrhetinic acid or derivatives thereof such as glycyrrhetinic acid, stearyl glycyrrhetinate and pyridoxine glycyrrhetinate; allantoin or derivatives thereof such as allantoin, allantoin β-glycyrrhetin and allantoin chlorohydroxyaluminum; indomethacin; ibuprofen; ibuprofen piconol; bufexamac; butyl flufenamate; bendazac; piroxicam; ketoprofen; felbinac; menthol; camphor; zinc oxide; azulenesulfonic acid, guaiazulenesulfonic acid; pyridoxine hydrochloride; lysozyme chloride; and the like.

### (Moisturizer)

Polyhydric alcohols such as polyethylene glycol, ethylene glycol, propylene glycol, 1,3-butylene glycol, glycerin, diethylene glycol, triethylene glycol, tetraethylene glycol, dipropylene glycol, diglycerin and polyethylene glycol;
glycol ethers such as ethylene glycol monomethyl ether, ethylene glycol monoethyl ether, ethylene glycol monopropyl ether, diethylene glycol monomethyl ether, diethylene glycol monoethyl ether, diethylene glycol monopropyl ether, diethylene glycol monobutyl ether, propylene glycol monoethyl ether, propylene glycol monopropyl ether, dipropylene glycol monoethyl ether and dipropylene glycol monopropyl ether;
sugar alcohols such as mannitol, sorbitol, erythritol, xylitol and trehalose;
phospholipids such as lecithin and hydrogenated lecithin;
high-molecular compounds such as diglycerin trehalose, heparin-like substances, collagen, elastin, keratin, chitin, chitosan, sodium hyaluronate, sodium acetyl hyaluronate and sodium chondroitin sulfate;
amino acid and derivatives thereof such as glycine, aspartic acid, arginine, alanine, serine, leucine, isoleucine, threonine, proline, hydroxyproline and theanine;
natural moisturizing factor (NMF)-derived components such as sodium lactate, urea and sodium pyrrolidone carboxylate;
plant extracts such as camomile extract, Aloe extract, Aloe vera extract, hamamelis extract, rosemary extract, thyme extract, tea extract and Perilla frutescens extract;
ceramides such as ceramide 1, ceramide 2, ceramide 3, ceramide 4, ceramide 5, ceramide 6I, ceramide 6II and ceramide 7;
N-(hexadecyloxyhydroxypropyl)-N-hydroxyethyl decanamide, N-(hexadecyloxyhydroxypropyl)-N-hydroxyethyl hexadecanamide and the like.

### (Vitamins)

Vitamins A such as retinol, retinol acetate, retinol palmitate, retinal, retinoic acid, methyl retinoate, ethyl retinoate, retinol retinoate, vitamin A fatty acid esters, d-δ-tocopheryl retinoate, α-tocopheryl retinoate, β-tocopheryl retinoate and vitamin A oil;
provitamins A such as β-carotene, α-carotene, γ-carotene, δ-carotene, lycopene, zeaxanthin, cryptoxanthin and echinenone;
vitamins E such as α-tocopherol, β-tocopherol, δ-tocopherol, tocopherol acetate, dl-α-tocopherol succinate and dl-α-tocopherol calcium succinate;
vitamins B2 such as riboflavin, flavin mononucleotide, flavin adenine dinucleotide, riboflavin butyrate, riboflavin tetrabutyrate, riboflavin 5'-phosphate sodium and riboflavin tetranicotinate;
nicotinic acids such as methyl nicotinate, nicotinic acid and nicotinamide;
vitamins C such as ascorbic stearate, L-ascorbyl dipalmitate, ascorbyl tetraisopalmitate, ascorbic acid, sodium ascorbate, dehydroascorbic acid, sodium ascorbic phosphate, magnesium ascorbic phosphate, magnesium ascorbic phosphate salt, sodium ascorbic phosphate salt and ascorbic acid glucoside;
vitamins D such as methyl hesperidin, ergocalciferol and cholecalciferol;
vitamins K such as phylloquinone and farnoquinone;
vitamins B1 such as γ-oryzanol, dibenzoyl thiamine, dibenzoyl thiamine hydrochloride, thiamine hydrochloride, thiamine cetyl hydrochloride, thiamine thiocyanate, thiamine lauryl hydrochloride, thiamine nitrate, thiamine monophosphate, lysine salt of thiamine, thiamine triphosphate, thiamine monophosphate phosphate salt, thiamine monophosphate, thiamine diphosphate, thiamine diphosphate hydrochloride, thiamine triphosphate and thiamine triphosphate monophosphate salt;
vitamins B6 such as pyridoxine hydrochloride, pyridoxine acetate, pyridoxal hydrochloride, pyridoxal 5'-phosphate and pyridoxamine hydrochloride;
vitamins B12 such as cyanocobalamin, hydroxocobalamin and deoxyadenosylcobalamin;
folic acids such as folic acid and pteroylglutamic acid;
pantothenic acids such as pantothenic acid, calcium pantothenate, pantothenyl alcohol (panthenol), D-pantetheine, D-pantethine, coenzyme A and pantothenyl ethyl ether;
biotins such as biotin and biocytin; and
vitamin-like acting factors such as carnitine, ferulic acid, α-lipoic acid and orotic acid;

### (Astringent)

Citric acid, tartaric acid, lactic acid, tannic acid, succinic acid, aluminum chloride, aluminum sulfate, allantoin chlorohydroxyaluminum, allantoin dihydroxyaluminum, aluminum phenolsulfonate, zinc para-phenolsulfonate, zinc sulfate, zinc lactate, aluminum chlorohydroxide, alum, chlorohydroxyaluminum, allantoin aluminum salt, potassium aluminum sulfate, dry aluminum hydroxide gel, aluminum glycinate and the like.

### (Anti-wrinkle agent)

Ubiquinones such as coenzymes Q6 to Q10, kinetin, glycolic acid, argiline, acylated glucosamine, collagen, sodium hyaluronate, sodium acetyl hyaluronate, aloe extract, seaweed extract, horse chestnut extract, rosemary extract, cornflower extract, and the like.

### <Method for preparing external composition for skin>

The method for preparing the external composition for skin of the present invention is not particularly limited, and the composition may be prepared by appropriately selecting the agent for giving warmth (A), the ingredient (B), and usual ingredients necessary for preparing external composition for skin (the above-described other ingredients) and mixing these ingredients in accordance with a conventional method. In addition, the dosage and usage of the external composition for skin of the present invention to integuments are not particularly limited. The composition may be usually used by applying, for example, coating, on integuments such as the skin several times a day each in an appropriate amount.

Examples of applications of the external composition for skin of the present invention include, but are not particularly limited to, medical applications and skin care applications. The external composition for skin of the present invention is preferably used for the purpose of giving warmth, relief of pain, anti-inflammatory effect, antipruritic effect and the like, particularly for the purpose of warming, relief of pain, anti-inflammatory effect and the like. Symptoms and diseases to which the external composition for skin of the present invention is applied include lumbago, back pain, arthritis, muscle tone, sprain and the like.

### [Pharmaceutical formulation of external composition for skin]

External preparations for skin can be prepared by forming the external composition for skin of the present invention to various pharmaceutical formulations. Examples of the pharmaceutical formulations include an ointment, liquid medicines (including an oil, a lotion, an emulsion and an aerosol), gels (including a liquid crystal, a microemulsion and liposome), adhesive skin patches (a cataplasm, a plaster (including a tape) and the like) and creams. These pharmaceutical formulations can be prepared by a conventionally known method. It is advantageous to form the external composition for skin of the present invention to a pharmaceutical formulation of an adhesive skin patch or a cream. The methods for forming the external composition for skin to these pharmaceutical formulations are described below.

As described above, the external composition for skin of the present invention contains the agent for giving warmth (A) and the ingredient (B) which is a specific ester compound. By inclusion of the ingredient (B), the warmth-giving effect of the agent for giving warmth (A) lasts for a long time. As a matter of course, regarding an external preparation for skin which contains the external composition for skin of the present invention, the warmth-giving effect of the agent for giving warmth (A) lasts for a long time. From another viewpoint, this means that the duration of the warmth-giving effect of the agent for giving warmth (A) can be extended by adding the ingredient (B) to an external preparation for skin which contains the agent for giving warmth (A).

As described above, it is advantageous to form the external composition for skin of the present invention to a pharmaceutical formulation of an adhesive skin patch or a cream. In particular, when the external preparation for skin of the present invention is used as an adhesive skin patch, the effect of the present invention that the duration of the warmth-giving effect is extended is significantly exhibited.

In addition, when the cream or adhesive skin patch contains as the ingredient (B) a fatty acid ester of an aliphatic polyhydric alcohol (excluding glyceryl tri-2-ethylhexanoate), the surface temperature can also be increased by using the cream or adhesive skin patch of the present invention.

That is, warmth can be continuously given for a long time by applying the adhesive skin patch or cream of the present invention, particularly the adhesive skin patch, to an affected part. As described above, when a specific compound is included as the ingredient (B), the surface temperature of the affected part can also be increased. The term "affected part" represents any part to which a various kinds of external preparation for skin of the present invention, including the adhesive skin patch and the cream of the present invention, may be applied, and is not limited to diseased or wounded parts. Parts to which the external preparation for skin of the present invention may be applied include a neck, a shoulder, an arm, a waist, a knee, an ankle and the like.

The adhesive skin patch and cream of the present invention are described below.

### <Adhesive skin patch>

Examples of the adhesive skin patch of the present invention include:
an adhesive skin patch (I) in which an adhesive layer containing the external composition for skin of the present invention is formed on a support; and
an adhesive skin patch (II) comprising a support, a layer (1) containing the external composition for skin of the present invention and an adhesive layer.

The adhesive layer of the adhesive skin patch (I) and the adhesive layer and the layer (1) of the adhesive skin patch (II) may contain the above-described other ingredients if necessary.
As an adhesive constituting the adhesive layer of the adhesive skin patches (I) and (II), any adhesive which is generally used for adhesive skin patches can be used without limitations, but an adhesive inducing little irritation on the skin is preferably used.

As the adhesive, mention may be made of a solvent-type, an emulsion-type and hot-melt type adhesive. Specific examples of such adhesives include adhesives containing:
an acrylic polymer and a salt thereof (for example, a (meth)acrylic acid homopolymer or copolymer, a (meth)acrylate homopolymer or copolymer, a (meth)acrylic acid/(meth)acrylate copolymer, or an alkali metal salt or alkaline-earth metal salt thereof);
a rubber-based polymer (natural rubber, isoprene rubber, polybutene, polyisobutylene, styrene/isoprene/styrene block copolymer, styrene/butadiene/styrene block copolymer, styrene-ethylene/butylene-styrene block copolymer, an alicyclic saturated hydrocarbon resin or the like);
a urethane polymer; and
a silicone polymer.
The adhesives may contain a known crosslinking agent (for example, an isocyanate or an aluminum compound).

The thickness of the adhesive layers of the adhesive skin patches (I) and (II) is generally about 400 to 3000 µm, preferably about 400 to 700 µm in the case of a cataplasm, and about 10 to 300 µm, preferably about 20 to 140 µm in the case of a tape. When the adhesive layers are excessively thin, the adhesive skin patches may be easily peeled due to insufficient adhesive force. On the other hand, when the adhesive layers are excessively thick, the adhesive skin patches may be easy to be physically peeled.

Regarding the adhesive skin patch (I), the content of the external composition for skin of the present invention in the adhesive layer is generally 0.01 to 80% by weight, preferably 0.1 to 70% by weight, more preferably 0.5 to 60% by weight based on the total (100% by weight) of the adhesive layer. When the content of the external composition for skin is less than 0.01% by weight, the satisfactory warmth effect may not be produced, while when the content exceeds 30% by weight, the satisfactory adhesive force of the adhesive layer may not be maintained.

Regarding the adhesive skin patch (II), the content of the external composition for skin of the present invention in the layer (1) is generally 0.01 to 99.9% by weight, preferably 0.1 to 99.9% by weight, more preferably 1 to 99.9% by weight based on the total (100% by weight) of the layer (1). When the content of the external composition for skin is less than 0.01% by weight, the satisfactory warmth effect may not be produced.

The support of the adhesive skin patches (I) and (II) is not particularly limited, and any support can be used as long as it has the ability to follow skin motions, i.e., flexibility. Specific examples thereof include a resin film, a nonwoven fabric, a cloth and a laminated product thereof.

As the resin film, a film composed of a known polymer can be used. Examples of the polymers include polyethylene, polypropylene, polyvinyl chloride, polyurethane, polyesters and ethylene-vinyl acetate copolymers. The resin film may be a single-layer film or a laminated film.

Examples of the nonwoven fabrics or clothes include, but are not particularly limited to, synthetic fibers such as polyethylene, polypropylene, polyurethane, polyesters and polyethylene terephthalate; and natural fibers such as cotton and silk. When the support is the nonwoven fabric or the cloth, these fibers may be used alone or used as a blend fabric of two or more kinds thereof.

The thickness of the support is generally about 30 to 2000 µm and preferably about 600 to 1300 µm. When the support is excessively thin, handleability of the adhesive skin patch may be degraded, thereby causing difficulty in application. When the support is excessively thick, the adhesive skin patch may be easily peeled or may cause physical irritation on the skin.

In addition, release paper may be laminated on the adhesive layer of the adhesive skin patch (I) and the layer of the adhesive skin patch (II) which is in direct contact with the affected part, in order to protect the adhesive layer or the like of the adhesive skin patch until its use. As the release paper, plastic films such as a polyethylene film or any other release papers usually used for adhesive skin patches can be used.

In the adhesive skin patch (I), an intermediate layer such as a porous film for controlling evaporation of moisture or an anchoring agent layer for fixing the adhesive may be formed between the support and the adhesive layer. Examples of the porous film include cellulose usually used for adhesive skin patches. Examples of the anchoring agent include acrylic polymers (e.g., (meth)acrylate homopolymer or copolymers).

In the adhesive skin patch (II), the support layer, the layer (1) and the adhesive layer are preferably laminated in this order.
The method for producing the adhesive skin patch (I) is not particularly limited, and a usual production method can be used. For example, the external composition for skin of the present invention and the adhesive, and, if required, the other ingredients are mixed and then the resultant mixture is applied and spread on the support or the intermediate layer formed on the support by a usual method to form the adhesive layer. If required, the release paper is laminated on the surface of the adhesive layer in order to protect the adhesive layer. Further, if required, the resultant product is cut into a predetermined size to produce the desired adhesive skin patch (I).

Also, the method for producing the adhesive skin patch (II) is not particularly limited and is basically the same as the production method for the adhesive skin patch (I) except that the adhesive layer and the layer (1) containing the external composition for skin of the present invention are separated. For example, a composition containing the external composition for skin of the present invention and, if required, the other ingredients is applied and spread on the support layer by a usual method to form the layer (1). Then, a composition containing the adhesive and, if required, the other ingredients is applied and spread on the layer (1) to form the adhesive layer. If required, the release paper is laminated on the surface of the adhesive layer in order to protect the adhesive layer. Further, if required, the resultant product is cut into a predetermined size to produce the desired adhesive skin patch (II).

Examples of the shapes of the adhesive skin patches (I) and (II) include a strip shape, a circular shape, an elliptic shape, a triangular shape, a boomerang shape and a facemask shape.

### <Cream>

The cream of the present invention is characterized in that it contains the external composition for skin of the present invention.

As specified in the Japanese Pharmacopoeia, the cream is an ointment using an emulsified base.
The content of the external composition for skin of the present invention in the cream is generally 0.01 to 80% by weight, preferably 0.1 to 70% by weight, and more preferably 0.5 to 60% by weight based on the total cream (100% by weight).

The method for producing the cream of the present invention is not particularly limited, and a usual production method can be used. For example, the external composition for skin of the present invention and the emulsified base, and, if required, the other ingredients are mixed to produce the cream.

### EXAMPLES

Although the present invention is described in detail below on the basis of examples, the present invention is not limited thereto.

### [EXAMPLE 1]

Capsaicinoid (the agent for giving warmth (A)) was dissolved in propylene glycol monocaprylate (ingredient (B), and the resultant solution was uniformly mixed with the other ingredients shown in Table 1 below to form a preparation. The preparation was uniformly spread on a cloth (nonwoven fabric made of polyethylene terephthalate, thickness 700 µm, manufactured by Japan Vilene Co., Ltd.) to form a layer of agent for giving warmth. Then, a plastic film was laminated on the layer of agent for giving warmth to complete an adhesive skin patch (cataplasm).

### [EXAMPLE 2]

An adhesive skin patch (cataplasm) was produced by the same method as in Example 1 except that glycol salicylate was used in place of propylene glycol monocaprylate.

### [COMPARATIVE EXAMPLES 1 to 7]

Adhesive skin patches (cataplasms) were produced by the same method as in Example 1 except that castor oil, sunflower oil, isopropyl myristate, diethyl sebacate, propylene glycol, 1,3-butylene glycol or glyceryl tri-2-ethylhexanoate was used in place of propylene glycol monocaprylate as shown in the below Table 1.

The thickness of the adhesive layer of the adhesive skin patch of each of Examples 1 and 2 and Comparative Examples 1 to 7 was 500 to 600 µm.

[Table 1]

**Table 1 Cataplasm**

| Ingredient | Example 1 | Example 2 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| Capsaicinoid*¹ | 0.04 | 0.04 | 0.04 | 0.04 | 0.04 | 0.04 | 0.04 | 0.04 | 0.04 |
| Glycerin | 20.00 | 20.00 | 20.00 | 20.00 | 20.00 | 20.00 | 20.00 | 20.00 | 20.00 |
| Polyacrylic acid | 2.00 | 2.00 | 2.00 | 2.00 | 2.00 | 2.00 | 2.00 | 2.00 | 2.00 |
| Partially neutralized polyacrylic acid | 6.00 | 6.00 | 6.00 | 6.00 | 6.00 | 6.00 | 6.00 | 6.00 | 6.00 |
| Sodium polyacrylate | 1.00 | 1.00 | 1.00 | 1.00 | 1.00 | 1.00 | 1.00 | 1.00 | 1.00 |
| Kaolin | 6.00 | 6.00 | 6.00 | 6.00 | 6.00 | 6.00 | 6.00 | 6.00 | 6.00 |
| Carmellose sodium | 3.00 | 3.00 | 3.00 | 3.00 | 3.00 | 3.00 | 3.00 | 3.00 | 3.00 |
| Tartaric acid | 1.00 | 1.00 | 1.00 | 1.00 | 1.00 | 1.00 | 1.00 | 1.00 | 1.00 |
| Polysorbate 80 | 0.50 | 0.50 | 0.50 | 0.50 | 0.50 | 0.50 | 0.50 | 0.50 | 0.50 |
| Dry aluminum hydroxide gel | 0.50 | 0.50 | 0.50 | 0.50 | 0.50 | 0.50 | 0.50 | 0.50 | 0.50 |
| Disodium edetate | 0.10 | 0.10 | 0.10 | 0.10 | 0.10 | 0.10 | 0.10 | 0.10 | 0.10 |
| Methyl paraoxybenzoate | 0.10 | 0.10 | 0.10 | 0.10 | 0.10 | 0.10 | 0.10 | 0.10 | 0.10 |
| Propylene glycol monocaprylate | 1.00 | - | - | - | - | - | - | - | - |
| Glycol salicylate | - | 1.00 | - | - | - | - | - | - | - |
| Castor oil | - | - | 1.00 | - | - | - | - | - | - |
| Sunflower oil | - | - | - | 1.00 | - | - | - | - | - |
| 1,3-butylene glycol | - | - | - | - | 1.00 | - | - | - | - |
| Propylene glycol | - | - | - | - | - | 1.00 | - | - | - |
| Isopropyl myristate | - | - | - | - | - | - | 1.00 | - | - |
| Diethyl sebacate | - | - | - | - | - | - | - | 1.00 | - |
| Glyceryl tri-2-ethylhexanoate | - | - | - | - | - | - | - | - | 1.00 |
| Purified water | 58.76 | 58.76 | 58.76 | 58.76 | 58.76 | 58.76 | 58.76 | 58.76 | 58.76 |
| Total | 100.00 | 100.00 | 100.00 | 100.00 | 100.00 | 100.00 | 100.00 | 100.00 | 100.00 |

| | | | | | | | | | |
|---|---|---|---|---|---|---|---|---|---|
| *1: Capsaicin USP, manufactured by Alps Pharmaceutical Co., Ltd. * In Table 1, the unit of numerical values is "parts by weight". | | | | | | | | | |

### [TEST EXAMPLE 1]

The adhesive skin patch of each of Examples 1 and 2 and Comparative Examples 1 to 7 was applied to the waists of 5 subjects for 8 hours. The subjects were made to evaluate warmth in accordance with the criteria below 0 minute (immediately after the application), 15 minutes, 30 minutes, 45 minutes, 60 minutes, 120 minutes, 240 minutes and 480 minutes after the application. The points of the five subjects were summed up to determine a warmth point.

0 point: No sensation or excessively strong warmth or irritating sensation/pain
1 point: Weak warmth
2 points: Strong warmth
3 points: Comfortable warmth.

The results are shown in Table 2 and illustrated in Fig. 1. Table 2 and Fig. 1 show the results on the basis of the ingredient (B). For example, in Table 2 and Fig. 1, "Castor oil" represents Comparative Example 1.

[Table 2]

**Table 2-1**

| Ingredient (B) | Subject No. | 0 min | 15 min | 30 min | 45 min | 60 min | 120 min | 240 min | 480 min |
|---|---|---|---|---|---|---|---|---|---|
| Castor oil | 1 | 0 | 0 | 1 | 3 | 2 | 0 | 0 | 0 |
| | 2 | 0 | 0 | 1 | 1 | 3 | 3 | 1 | 0 |
| | 3 | 0 | 0 | 1 | 3 | 2 | 3 | 1 | 0 |
| | 4 | 0 | 0 | 0 | 1 | 1 | 1 | 1 | 0 |
| | 5 | 0 | 0 | 1 | 1 | 3 | 3 | 1 | 0 |
| Total | | 0 | 0 | 4 | 9 | 11 | 10 | 4 | 0 |
| Sunflower oil | 1 | 0 | 0 | 1 | 1 | 3 | 1 | 0 | 0 |
| | 2 | 0 | 0 | 1 | 1 | 3 | 1 | 0 | 0 |
| | 3 | 0 | 0 | 0 | 1 | 1 | 1 | 0 | 0 |
| | 4 | 0 | 0 | 1 | 1 | 1 | 1 | 0 | 0 |
| | 5 | 0 | 0 | 1 | 3 | 2 | 2 | 0 | 0 |
| Total | | 0 | 0 | 4 | 7 | 10 | 6 | 0 | 0 |
| Isopropyl myristate | 1 | 0 | 0 | 0 | 0 | 1 | 1 | 0 | 0 |
| | 2 | 0 | 0 | 0 | 1 | 3 | 1 | 0 | 0 |
| | 3 | 0 | 0 | 0 | 1 | 3 | 1 | 1 | 1 |
| | 4 | 0 | 0 | 0 | 0 | 1 | 1 | 1 | 1 |
| | 5 | 0 | 0 | 0 | 0 | 1 | 1 | 1 | 0 |
| Total | | 0 | 0 | 0 | 2 | 9 | 5 | 3 | 2 |
| Diethyl sebacate | 1 | 0 | 0 | 0 | 1 | 1 | 1 | 0 | 0 |
| | 2 | 0 | 0 | 0 | 3 | 2 | 3 | 1 | 0 |
| | 3 | 0 | 0 | 0 | 1 | 1 | 1 | 1 | 1 |
| | 4 | 0 | 0 | 0 | 1 | 1 | 3 | 1 | 0 |
| | 5 | 0 | 0 | 0 | 3 | 2 | 1 | 0 | 0 |
| Total | | 0 | 0 | 0 | 9 | 7 | 9 | 3 | 1 |
| Propylene glycol | 1 | 0 | 0 | 0 | 1 | 1 | 1 | 0 | 0 |
| | 2 | 0 | 0 | 1 | 1 | 3 | 2 | 3 | 1 |
| | 3 | 0 | 0 | 1 | 1 | 3 | 1 | 0 | 1 |
| | 4 | 0 | 0 | 0 | 1 | 1 | 1 | 1 | 0 |
| | 5 | 0 | 0 | 0 | 1 | 1 | 1 | 0 | 0 |
| Total | | 0 | 0 | 2 | 5 | 9 | 6 | 4 | 2 |

**Table 2-2**

| Ingredient (B) | Subject No. | 0 min | 15 min | 30 min | 45 min | 60 min | 120 min | 240 min | 480 min |
|---|---|---|---|---|---|---|---|---|---|
| 1,3-Butylene glycol | 1 | 0 | 0 | 0 | 1 | 3 | 1 | 0 | 0 |
| | 2 | 0 | 0 | 0 | 3 | 3 | 1 | 0 | 0 |
| | 3 | 0 | 0 | 0 | 0 | 1 | 1 | 1 | 0 |
| | 4 | 0 | 0 | 0 | 0 | 1 | 1 | 0 | 0 |
| | 5 | 0 | 0 | 0 | 0 | 0 | 1 | 0 | 0 |
| Total | | 0 | 0 | 0 | 4 | 8 | 5 | 1 | 0 |
| Glycol salicylate | 1 | 0 | 1 | 2 | 2 | 2 | 2 | 3 | 3 |
| | 2 | 0 | 1 | 2 | 2 | 2 | 2 | 1 | 1 |
| | 3 | 0 | 0 | 1 | 2 | 3 | 3 | 1 | 1 |
| | 4 | 0 | 1 | 2 | 2 | 3 | 1 | 1 | 1 |
| | 5 | 0 | 0 | 1 | 3 | 2 | 3 | 3 | 1 |
| Total | | 0 | 3 | 8 | 11 | 12 | 11 | 9 | 7 |
| Propylene glycol monocaprylate | 1 | 0 | 1 | 3 | 3 | 2 | 2 | 3 | 3 |
| | 2 | 0 | 1 | 3 | 3 | 3 | 3 | 3 | 1 |
| | 3 | 0 | 1 | 3 | 3 | 3 | 3 | 3 | 1 |
| | 4 | 0 | 1 | 3 | 2 | 3 | 3 | 3 | 3 |
| | 5 | 0 | 1 | 3 | 2 | 2 | 3 | 3 | 3 |
| Total | | 0 | 5 | 15 | 13 | 13 | 14 | 15 | 11 |
| Glyceryl tri-2-ethylhexanoate | 1 | 0 | 0 | 0 | 1 | 1 | 1 | 0 | 0 |
| | 2 | 0 | 1 | 2 | 3 | 3 | 0 | 0 | 0 |
| | 3 | 0 | 0 | 0 | 0 | 1 | 1 | 1 | 0 |
| | 4 | 0 | 0 | 1 | 3 | 1 | 0 | 0 | 0 |
| | 5 | 0 | 0 | 2 | 3 | 1 | 1 | 0 | 0 |
| Total | | 0 | 1 | 5 | 10 | 7 | 3 | 1 | 0 |

Comparative Example 7 (glyceryl tri-2-ethylhexanoate as the ingredient (B)) has a composition close to Example 1 of Patent Document 2. Table 2 and Fig. 1 indicate that its warmth-giving effect does not last, which is similar to the cases in the other comparative examples. In contrast, it is found that in Examples 1 and 2 relating to the adhesive skin patches of the present invention, the good warmth-giving effect lasts for a long time without causing irritating sensation or pain.

### [TEST EXAMPLE 2]

The adhesive skin patch of each of Examples 1 and 2 and Comparative Examples 1, 5 and 7 was applied to the insides of the forearms of 6 subjects for 6 hours, and the temperatures of the skin surface on the applied parts were measured 2 hours, 4 hours and 6 hours after the application. The temperature of the skin surface before the application was measured and regarded as a reference.

The temperature of the skin surface was measured with a radiation thermometer (IR-0506C; manufactured by Minolta Co., Ltd.). The measurement results are shown in Table 3 and Fig. 2. The measurement results are shown by degree changes from the reference temperature. Table 3 and Fig. 2 show the results on the basis of the ingredient (B). For example, in Table 3 and Fig. 2, "Castor oil" represents Comparative Example 1.

[Table 3]

**Table 3**

| | Temperature change (°C) at respective times (hours) after application | | | |
|---|---|---|---|---|
| Ingredient (B) | 0 hour | 2 hours | 4 hours | 6 hours |
| Castor oil | 0 | -0.22 | -0.73 | -0.05 |
| Isopropyl myristate | 0 | -0.20 | -0.58 | 0.03 |
| Glycol salicylate | 0 | -0.17 | -0.15 | 0.38 |
| Glyceryl tri-2-ethylhexanoate | 0 | -0.35 | -0.28 | 0.67 |
| Propylene glycol monocaprylate | 0 | 0.30 | 0.28 | 0.80 |

In Example 1 (propylene glycol monocaprylate), an increase in skin surface temperature was observed 2 hours after the application, and the effect of increasing the skin surface temperature lasted 4 hours and 6 hours after the application.

### [Formulation Example]

Although formulation examples are described below, the present invention is not limited to these examples. In the formulation examples, cataplasms, plasters and creams were prepared by usual methods.

[Table 4]

**Table 4 Cataplasm**

| Ingredient | Formulation Example 1 | Formulation Example 2 | Formulation Example 3 | Formulation Example 4 | Formulation Example 5 | Formulation Example 6 | Formulation Example 7 | Formulation Example 8 | Formulation Example 9 |
|---|---|---|---|---|---|---|---|---|---|
| Capsaicinoid*¹ | 0.04 | - | - | 0.10 | - | - | 0.10 | - | - |
| Capsicum extract | - | 0.30 | - | - | 0.50 | - | - | 0.50 | - |
| Nonylic acid vanillylamide | - | - | 0.03 | - | - | 0.05 | - | - | 0.05 |
| Glycerin | 20.00 | 20.00 | 20.00 | 20.00 | 20.00 | 20.00 | 20.00 | 20.00 | 20.00 |
| Polyacrylic acid | 2.00 | 2.00 | 2.00 | 2.00 | 2.00 | 2.00 | 2.00 | 2.00 | 2.00 |
| Partially neutralized polyacrylic acid | 5.00 | 5.00 | 5.00 | 5.00 | 5.00 | 5.00 | 5.00 | 5.00 | 5.00 |
| Sodium polyacrylate | 1.00 | 1.00 | 1.00 | 1.00 | 1.00 | 1.00 | 1.00 | 1.00 | 1.00 |
| Talc | 6.00 | 6.00 | 6.00 | 6.00 | 6.00 | 6.00 | 6.00 | 6.00 | 6.00 |
| Polyvinyl alcohol | 1.00 | 1.00 | 1.00 | 1.00 | 1.00 | 1.00 | 1.00 | 1.00 | 1.00 |
| Sorbitol solution (70%) | 10.00 | 10.00 | 10.00 | 10.00 | 10.00 | 10.00 | 10.00 | 10.00 | 10.00 |
| Tartaric acid | 0.50 | 0.50 | 0.50 | 0.50 | 0.50 | 0.50 | 0.50 | 0.50 | 0.50 |
| Polysorbate 80 | 0.50 | 0.50 | 0.50 | 0.50 | 0.50 | 0.50 | 0.50 | 0.50 | 0.50 |
| Aluminum glycinate | 1.00 | 1.00 | 1.00 | 1.00 | 1.00 | 1.00 | 1.00 | 1.00 | 1.00 |
| Disodium edetate | 0.10 | 0.10 | 0.10 | 0.10 | 0.10 | 0.10 | 0.10 | 0.10 | 0.10 |
| Methyl paraoxybenzoate | 0.10 | 0.10 | 0.10 | 0.10 | 0.10 | 0.10 | 0.10 | 0.10 | 0.10 |
| Castor oil | 1.00 | 1.00 | 1.00 | 3.00 | - | - | - | - | - |
| Isopropyl myristate | - | - | - | - | 2.00 | - | - | - | - |
| 1-Menthol | 3.00 | 2.00 | 1.00 | - | - | - | - | - | - |
| Ibuprofen | 5.00 | 3.00 | - | - | 5.00 | 3.00 | - | - | - |
| Glycol salicylate | - | - | - | - | - | 1.00 | - | - | - |
| Methyl salicylate | - | - | 10.00 | - | - | - | - | - | - |
| Propylene glycol monocaprylate | 1.00 | | - | 3.00 | - | - | 5.00 | - | - |
| Propylene glycol dicaprylate | - | 1.00 | - | - | 3.00 | - | - | 5.00 | - |
| Propylene glycol didecanoate | - | - | 1.00 | - | - | 3.00 | - | - | 5.00 |
| Purified water | 42.76 | 45.50 | 39.77 | 46.70 | 42.30 | 45.75 | 47.70 | 47.30 | 47.75 |
| Total | 100.00 | 100.00 | 100.00 | 100.00 | 100.00 | 100.00 | 100.00 | 100.00 | 100.00 |

| | | | | | | | | | |
|---|---|---|---|---|---|---|---|---|---|
| *1: Capsaicin USP, manufactured by Alps Pharmaceutical Co., Ltd. * In Table 4, the unit of numerical values is "parts by weight". | | | | | | | | | |

**[Table 5] Plaster**

| Ingredient | Formulation Example 10 | Formulation Example 11 | Formulation Example 12 | Formulation Example 13 | Formulation Example 14 |
|---|---|---|---|---|---|
| Capsaicinoid^{*1} | - | - | 0.04 | - | - |
| Capsicum extract | - | 0.2 | - | 0.2 | 0.2 |
| Nonylic acid vanillylamide | 0.025 | - | - | - | |
| Styrene/isoprene/styrene block copolymer | 21.00 | 21.00 | 21.00 | 21.00 | 21.00 |
| Alicyclic saturated hydrocarbon resin | 40.00 | 40.00 | 40.00 | 40.00 | 40.00 |
| Polybutene | 10.00 | 10.00 | 10.00 | 10.00 | 10.00 |
| Liquid paraffin | 26.975 | 27.80 | 27.96 | 21.80 | 26.80 |
| Dibutylhydroxytoluene | 1.00 | 1.000 | 1.00 | 1.00 | 1.00 |
| Glycol salicylate | - | - | - | 5.00 | 1.00 |
| Propylene glycol monocaprylate | 1.00 | - | - | 1.00 | - |
| Propylene glycol dicaprylate | - | 1.00 | - | - | - |
| Propylene glycol didecanoate | - | - | 1.00 | - | 1.00 |
| Total | 100.00 | 100.00 | 100.00 | 100.00 | 100.00 |

| | | | | | |
|---|---|---|---|---|---|
| *1: Capsaicin USP, manufactured by Alps Pharmaceutical Co., Ltd. * In Table 5, the unit of numerical values is "parts by weight". | | | | | |

[Table 6]

**Table 6 Cream**

| Ingredient | Formu. Ex. 15 | Formu. Ex. 16 | Formu. Ex. 17 | Formu. Ex. 18 | Formu. Ex. 19 |
|---|---|---|---|---|---|
| Methyl salicylate | 12.00 | 12.00 | 7.00 | 12.00 | 7.50 |
| 1-Menthol | 6.00 | 6.00 | 6.00 | 6.00 | 6.00 |
| Eucalyptus oil | 2.00 | - | - | 2.00 | - |
| Turpentine oil | 1.50 | - | - | 1.50 | - |
| dl-camphor | - | 2.00 | 2.00 | - | 2.00 |
| Nonylic acid vanillylamide | 0.01 | 0.01 | 0.01 | 0.01 | 0.01 |
| Propylene glycol monocaprylate | 1.00 | 1.00 | 1.00 | - | - |
| Isopropyl palmitate | 5.00 | - | - | 5.00 | - |
| Isopropyl myristate | - | 5.00 | - | - | 5.00 |
| Glycol salicylate | - | - | 5.00 | - | - |
| Cetanol | 3.00 | 3.00 | 3.00 | 3.00 | 3.00 |
| Glyceryl monostearate | 15.00 | 15.00 | 15.00 | 15.00 | 15.00 |
| Polyoxyethylene hydrogenated castor oil | 3.00 | 3.00 | 3.00 | 3.00 | 3.00 |
| Methyl paraoxybenzoate | 0.10 | 0.10 | 0.10 | 0.10 | 0.10 |
| Sodium edetate hydrate | 0.05 | 0.05 | 0.05 | 0.05 | 0.05 |
| Carboxyvinyl polymer | 0.50 | 0.50 | 0.50 | 0.50 | 0.50 |
| Triethanolamine | 0.50 | 0.50 | 0.50 | 0.50 | 0.50 |
| Purified water | 50.34 | 51.84 | 56.84 | 51.34 | 57.34 |

| | | | | | |
|---|---|---|---|---|---|
| * In Table 6, the unit of numerical values is "parts by weight". | | | | | |

## Claims

1. An external composition for skin comprising:
(A) an agent for giving warmth; and
(B) at least one selected from the group consisting of fatty acid esters of aliphatic polyhydric alcohols (excluding glyceryl tri-2-ethylhexanoate) and aromatic carboxylic acid esters of lower alcohols or aliphatic polyhydric alcohols.

2. The external composition for skin according to Claim 1, wherein the agent for giving warmth (A) contains at least one selected from the group consisting of capsaicinoid, N-acylvanillylamide and vanillyl alcohol alkyl ethers.

3. The external composition for skin according to Claim 1, wherein the ingredient (B) contains an aliphatic polyhydric alcohol fatty acid ester (excluding glyceryl tri-2-ethylhexanoate).

4. The external composition for skin according to Claim 1, wherein the ingredient (B) contains a propylene glycol fatty acid ester.

5. The external composition for skin according to Claim 1, wherein the ingredient (B) contains at least one selected from the group consisting of propylene glycol monocaprylate, propylene glycol dicaprylate and propylene glycol didecanoate.

6. The external composition for skin according to any one of Claims 1 to 5, wherein the agent for giving warmth (A) contains capsaicinoid.

7. An external preparation for skin comprising the external composition for skin according to any one of Claims 1 to 6.

8. An adhesive skin patch in which an adhesive layer containing the external composition for skin according to any one of Claims 1 to 6 is formed on a support.

9. An adhesive skin patch comprising a support, a layer (1) containing the external composition for skin according to any one of Claims 1 to 6 and an adhesive layer.

10. The adhesive skin patch according to Claim 9, wherein the support, the layer (1) and the adhesive layer are laminated in this order.

11. A cream comprising the external composition for skin according to any one of Claims 1 to 6.

12. A method for continuously giving warmth to an affected part, comprising applying the adhesive skin patch according to any one of Claims 8 to 10 or the cream according to Claim 11 to the affected part.

13. Use of the adhesive skin patch according to any one of Claims 8 to 10 or the cream according to Claim 11 for continuously giving warmth to an affected part.

14. The adhesive skin patch according to any one of Claims 8 to 10 or the cream according to Claim 11, which is used for continuously giving warmth to an affected part.

15. A method for continuously giving warmth by using the followings in combination:
(A) an agent for giving warmth; and
(B) at least one selected from the group consisting of fatty acid esters of aliphatic polyhydric alcohols (excluding glyceryl tri-2-ethylhexanoate) and aromatic carboxylic acid esters of lower alcohols or aliphatic polyhydric alcohols.

16. A method for extending the duration of warmth giving- action of an agent for giving warmth (A), comprising:
adding, to an external preparation for skin which contains the agent for giving warmth (A), at least one selected from the group consisting of fatty acid esters of aliphatic polyhydric alcohols (excluding glyceryl tri-2-ethylhexanoate) and aromatic carboxylic acid esters of lower alcohols or aliphatic polyhydric alcohols.
